# EUROPÄISCHE PATENTSCHRIFT

(11) **EP 0 276 775 B1**
(45) Veröffentlichungstag und Bekanntmachung des Hinweises auf die Patenterteilung: **03.03.1993**
(21) Anmeldenummer: 88100904.7
(22) Anmeldetag: 22.01.1988
(51) Int. Cl.: C07C 21/06, C07C 17/34

(54) **Verfahren zur Herstellung von Vinylchlorid durch thermische Spaltung von 1,2-Dichlorethan**
Method for the production of vinyl chloride by pyrolysis of 1,2-dichloroethane
Procédé pour la fabrication de chlorure de vinyle par pyrolyse d'1,2-dichloréthane

(30) Priorität: 28.01.1987 DE 3702438
(43) Veröffentlichungstag der Anmeldung: 03.08.1988
(73) Patentinhaber: HOECHST AKTIENGESELLSCHAFT, 65926 Frankfurt am Main (DE); Uhde GmbH, 44141 Dortmund (DE)
(72) Erfinder: Link, Gerhard, D-6500 Mainz (DE); Fröhlich, Walter, Dr., D-8269 Burgkirchen (DE); Krumböck, Reinhard, D-8269 Burgkirchen (DE); Prantl, Georg, D-8263 Burghausen (DE); Schaffelhofer, Iwo, Dr., D-8263 Burghausen (DE)

(56) Entgegenhaltungen:
- EP-A- 0 014 920
- EP-A- 0 021 381
- EP-A- 0 180 925
- EP-A- 0 180 995
- DE-A- 2 913 030
- GB-A- 2 179 938
- PATENT ABSTRACTS OF JAPAN, Band 6, n3. 239 (C-137)(1117), 26. November 1982; & JP-A-57 142 928

## Beschreibung

Die Erfindung betrifft ein Verfahren zur Herstellung von Vinylchlorid gemäß Anspruch 1.

Die unvollständige thermische Spaltung von 1,2-Dichlorethan bei Ofen-Eingangsdrucken von 0,8 bis 4 MPa und Temperaturen von 450 bis 550 °C zur Gewinnung von Vinylchlorid wird seit vielen Jahren großtechnisch betrieben. Das Verfahren benötigt zum Erhitzen des 1,2-Dichlorethans bis zur Spalttemperatur, zur anschließenden destillativen Auftrennung des Spaltgas-Gemisches sowie zur Reinigung des nicht-umgesetzten und des neu zugeführten 1,2-Dichlorethans beträchtliche Energiemengen, die einen wesentlichen Kostenfaktor darstellen. Zur Rückgewinnung eines Teils der in der verschiedenen Verfahrensschritten aufgewendeten Energie gibt es eine Reihe bekannter Verfahren, die sich teilweise auf die Destillation, teilweise auf die bei der thermischen Spaltung des 1,2-Dichlorethans aufgewendete Energie beziehen. Mit diesen Versfahren kann der Herstellungsprozeß von Vinylchlorid deutlich günstiger bezüglich des Energieverbrauches gestaltet werden, wobei abhängig von der Art des verwendeten, energetisch recht komplexen Prozesses mal diese, mal jene Verfahren günstiger anzuwenden sind. In jedem Fall ist es von Vorteil, eine Vielzahl von Verfahren zur Energie-Rückgewinnung an der Hand zu haben, um durch entsprechende Auswahl den jeweiligen Prozeß optimieren zu können.

Zur Wiederverwendung der in den heißen vinylchloridhaltigen Gasen, die den Spaltofen verlassen, enthaltenen Energie sind aus den europäischen Patentanmeldungen 14 920 und 21 381 Verfahren bekannt, diese Gase zu benutzen, um beispielsweise Wasser oder 1,2-Dichlorethan zu verdampfen und den Wasserdampf zur Heizung von Apparaturen an anderer Stelle des Vinylchlorid-Herstellungsprozesses zu verwenden bzw. das verdampfte 1,2-Dichlorethan mit einer Temperatur von 240 bis 260 °C der Spaltzone des Pyrolyseofens zuzuführen. Nach dem Verfahren der EP 180 995 wird das den Spaltofen verlassende Gasgemisch durch direkte Kühlung abgeschreckt und einer Quenchkolonne aufgegeben, in der das Gasgemisch als Brüden gewonnen wird. Diese Brüden werden durch indirekte Kühlung bis mindestens auf ihren Taupunkt gebracht und mit der abgegebenen Wärme, gemäß dem Beispiel, flüssiges 1,2-Dichlorethan auf 80 °C und Verbrennungsluft für den Splatofen auf 110 °C erwärmt sowie der Sumpf der Destillationskolonne zur Abtrennung des Chlorwasserstoffs geheizt.

In neuerer Zeit wurde vorgeschlagen (DE-Patentanmeldung P 36 34 550.4), mit den heißen vinylchloridhaltigen Gasen aus dem Spaltofen flüssiges 1,2-Dichlorethan in einer Apparatur, bestehend aus zwei Behältern, zu verdampfen und ohne weitere Erwärmung mit einer Temperatur von 170 bis 280 °C in den Spaltofen einzuspeisen.

Alle diese bekannten Verfahren benutzen die in den Spaltgasen enthaltene Wärme im wesentlichen, um flüssiges 1,2-Dichlorethan zu erwärmen und zu verdampfen.

Es wurde nun ein Verfahren gefunden, das es ermöglicht, gasförmiges 1,2-Dichlorethan, das beispielsweise durch Sieden bei 170 bis 280 °C gewonnen wurde, weiter bis in die Nähe der Spalttemperatur zu erwärmen, wobei partielle Überhitzungen, wie sie bei der bisher üblichen Erwärmung in der Strahlungszone des Spaltofens vorkommen, vermieden werden.

Das neue Verfahren zur Herstellung von Vinylchlorid durch thermische Abspaltung von Chlorwasserstoff aus 1,2-Dichlorethan in einem Spaltofen, der eine Strahlungszone enthält, wobei 1,2-Dichlorethan in einem Wärmetauscher mit heißem, Vinylchlorid enthaltendem Gas, das nach der thermischen Spaltung aus der Strahlungszone des Spaltofens abgeführt wird, indirekt erwärmt wird, ist dadurch gekennzeichnet, daß ein Gas, das mindestens 95 Gew.-% 1,2-Dichlorethan enthält, von 170 bis 280 °C auf eine Temperatur erwärmt wird, die mindestens 50 °C unter der Temperatur liegt, mit der das Vinylchlorid enthaltende Gas aus der Strahlungszone des Spaltofens abgeführt wird und anschließend das mindestens 95 Gew.-% 1,2-Dichlorethan enthaltende Gas in den Spaltofen eingespeist wird.

Als Strahlungszone wird der Kürze halber die Zone des Spaltofens bezeichnet, in der im wesentlichen die thermische Spaltung des 1,2-Dichlorethans zu Vinylchlorid und Chlorwasserstoff stattfindet. Zu einem Teil erfolgt in dieser Zone bei der derzeit üblichen Betriebsweise auch die Erwärmung des gasförmigen 1,2-Dichlorethans bis auf Spalttemperatur. Der Name "Strahlungszone" kommt daher, daß bei den bisher üblichen Spaltöfen in dieser Zone das Rohr, in dem das 1,2-Dichlorethan geführt wird, der direkten Wärmestrahlung durch die Brennerflammen, die den Ofen beheizen, ausgesetzt ist. Der in der Regel über der Strahlungszone liegende Teil des Ofens, in welchem das 1,2-Dichlorethan enthaltende Rohr nur noch durch die heißen Rauchgase der Brennerflammen beheizt wird, ist nachfolgend mit "Konvektionszone" bezeichnet.

Prinzipiell kann auch ein Gas, das weniger als 95 Gew.-% 1,2-Dichlorethan enthält, erfindungsgemäß behandelt werden, insbesondere wenn der Rest im wesentlichen aus Inertgas besteht. Wenn jedoch der Rest die prozeßüblichen Verunreinigungen sind, ist ein Gas mit weniger als 95 Gew.-% 1,2-Dichlorethan im allgemeinen für die thermische Spaltung zu Vinylchlorid ungeeignet. Diese Grenze wurde hier gewählt zum Unterschied von dem Gasgemisch, das nach der thermischen Spaltung den Spaltofen verläßt und noch beträchtliche Mengen nicht-umgesetztes 1,2-Dichlorethan, jedoch nicht mindestens 95 Gew.-%, enthält. Vorzugsweise wird ein mindestens 98 Gew.-% 1,2-Dichlorethan enthaltendes Gas erwärmt.

Das Gas, das nach der thermischen Spaltung aus der Strahlungszone des Spaltofens abgeführt wird, enthält in der Regel mindestens 20 Gew.-% Vinylchlorid. Darunter wird im allgemeinen die Durchführung des Spaltprozesses zu unwirtschaftlich. Nach oben ist der Vinylchlorid-Gehalt durch den erzielbaren Spaltumsatz begrenzt und wird im allgemeinen 50 Gew.-% nicht überschreiten.

Als Wärmetauscher sind Gegenstrom- oder Kreuzstrom-Wärmetauscher üblicher Bauart geeignet, beispielsweise Doppelmantel-; Rohrbündel-; Spiral-; oder Rippenrohr-Wärmetauscher aus legierten chemisch beständigen Stählen. Auf welcher Seite der jeweiligen Wärmeaustauschfläche die beiden Gase strömen ist unwesentlich. Eine gewisse Mindestströmungsgeschwindigkeit der Gase, die von der Bauart des Wärmetauschers abhängt, sollte im Interesse eines guten Wärmeübergangs eingehalten werden. Im allgemeinen wird man eine Strömungsgeschwindigkeit von 8 m/s nicht unterschreiten.

Der Druck, unter dem das Vinylchlorid enthaltende Gas aus der Strahlungszone des Spaltofens abgeführt wird, ist für die Durchführbarkeit des Verfahrens nicht wesentlich. Aus wirtschaftlichen Erwägungen wird ein Druck von 0,5 bis 3 MPa bevorzugt gewählt, da unter 0,5 MPa die Raum-Zeit-Ausbeute verhältnismäßig gering wird und über 3 MPa aufwendige Hochdruck-Apparaturen verwendet werden müßten. Besonders gute Ergebnisse werden im Druckbereich von 1,6 bis 2,6 MPa erhalten.
Das Vinylchlorid enthaltende Gas wird im allgemeinen mit einer Temperatur von 450 bis 550 °C aus der Strahlungszone des Spaltofens abgeführt und in den Gas-Wärmetauscher eingeleitet. In diesem Wärmetauscher wird das mindestens 95 Gew.-% 1,2-Dichlorethan enthaltende Gas von 170 bis 280 °C, insbesondere von 230 bis 270 °C, vorteilhaft auf eine Temperatur erwärmt, die mindestens 50 °C unter der Temperatur liegt, mit der das Vinylchlorid enthaltende Gas aus der Strahlungszone des Spaltofens abgeführt wird. Bevorzugt wird das gasförmige 1,2-Dichlorethan auf eine Temperatur erwärmt, die mindestens 100 °C unter der Temperatur liegt, mit der das Vinylchlorid enthaltende Gas die Strahlungszone des Spaltofens verläßt.
Das erfindungsgemäße Verfahren kann in verschiedenen bevorzugten Varianten durchgeführt werden. Bei einer dieser Varianten wird ein Spaltofen verwendet, der eine Strahlungs- und eine Konvektionszone enthält. Das mindestens 95 Gew.-% 1,2-Dichlorethan enthaltende Gas wird ganz oder teilweise in der Konvektionszone des Spaltofens durch indirekten Wärmetausch mit den Rauchgasen aus flüssigem 1,2-Dichlorethan erzeugt.
Das in der Konvektionszone verdampfte 1,2-Dichlorethan wird aus dem Spaltofen abgeführt und außerhalb in einem Wärmetauscher mit den heißen Vinylchlorid enthaltenden Gasen erwärmt, anschließend in die Strahlungszone des Spaltofens eingeführt und dort die Hauptmenge des 1,2-Dichlorethans thermisch gespalten.
In einer weiteren Variante des erfindungsgemäßen Verfahrens wird das mindestens 95 Gew.-% 1,2-Dichlorethan enthaltende Gas ganz oder teilweise außerhalb des Spaltofens in einem Verdampfer aus flüssigem 1,2-Dichlorethan erzeugt. Als Heizmedien können hier verwendet werden: heiße Rauchgase, die aus einem Verbrennungsprozeß außerhalb des Spaltofens stammen, Hochdruck-Wasserdampf oder hochsiedende Flüssigkeiten wie Diphenyl, ^{®}Marlotherm S, kondensierte Aromaten oder ensprechend wärmebeständige Siliconöle, die auf die zur Verdampfung des 1,2-Dichlorethans notwendige Temperatur aufgeheizt wurden, beispielsweise durch einen Verbrennungsprozeß oder durch heiße Vinylchlorid enthaltende Gase, die nach der thermischen Spaltung des 1,2-Dichlorethans aus der Strahlungszone des Spaltofens geführt wurden. Sofern diese Gase erfindungsgemäß zunächst zur Aufheizung von gasförmigem 1,2-Dichlorethan verwendet wurden, können sie immer noch anschließend zur Vorheizung dieser Wärmeübertragungsmedien dienen.

In einer weiteren bevorzugten Ausführungsform des erfindungsgemäßen Verfahrens wird das mindestens 95 Gew.-% 1,2-Dichlorethan enthaltende Gas ganz oder teilweise aus flüssigem 1,2-Dichlorethan durch indirekten Wärmetausch mit heißem Vinylchlorid enthaltendem Gas, das nach der thermischen Spaltung des 1,2-Dichlorethans aus der Strahlungszone des Spaltofens abgefüht wurde, erzeugt. Hierfür können beispielsweise die weiter oben erwähnten Verfahren nach den europäischen Patentanmeldungen 14 920 und 21 381 sowie auch das in neuerer Zeit vorgeschlagene Verfahren nach der DE-Patentanmeldung P 36 34 550.4 verwendet werden. Besonders vorteilhaft ist die Hintereinanderschaltung des erfindungsgemäßen Verfahrens und eines Verfahrens, wie es in den im vorigen Satz genannten Patentanmeldungen beschrieben ist. Hierbei wird zunächst das heiße, Vinylchlorid enthaltende Gas dazu benutzt, um erfindungsgemäß gasförmiges 1,2-Dichlorethan aufzuheizen und anschließend um flüssiges 1,2-Dichlorethan zu verdampfen. Das nach dieser Verdampfung anfallende, immer noch mindestens 170 bis 280 °C warme Vinylchlorid enthaltende Gas kann anschließend vorteilhaft in einem weiteren Wärmeaustauscher dazu benutzt werden, flüssiges 1,2-Dichlorethan bis in die Nähe der Verdampfungstemperatur zu erwärmen. Dann wird das Vinylchlorid enthaltende Gas zur Kondensation des darin enthaltenen nicht-umgesetzten 1,2-Dichlorethans und des Vinylchlorids sowie weiterer Nebenbestandteile auf -20 bis -50 °C abgekühlt und wie üblich in einer Kolonne der gasförmige Chlorwasserstoff abgetrennt. Der Druck am Kopf dieser Kolonne wird in bekannter Weise vorteilhaft so eingestellt, daß das heiße Vinylchlorid enthaltende Gas die Strahlungszone des Spaltofens mit eine Druck von 0,5 bis 3 MPa verläßt.

Insbesondere wenn das erfindungsgemäße Verfahren mit einer Verdampfung des flüssigen 1,2-Dichlorethans zur Erzeugung eines mindestens 95 Gew.-% 1,2-Dichlorethan enthaltenden Gases außerhalb des Spaltofens benutzt wurde, kann das mindestens 95 Gew.-% 1,2-Dichlorethan enthaltende Gas nach der erfindungsgemäßen Aufheizung sowohl in die Strahlungszone als auch in die Konvektionszone des Spaltofens eingeleitet werden.

Das mindestens 95 Gew.-% 1,2-Dichlorethan enthaltende Gas kann aus einer Destillationskolonne stammen, nach der es mit üblichen Verdichtern auf den Druck gebracht wird, der zur Einspeisung in den Spaltofen nach der erfindungemäßen Erwärmung notwendig ist.

Wie eingangs bereits erwähnt, ermöglicht es das erfindungsgemäße Verfahren, die in den heißen Gasen, die den Spaltofen verlassen, enthaltene Wärme auszunutzen, wobei gegenüber den bekannten Verfahren eine zusätzliche Einsparung an Primärenergie (Brennstoff) eintritt. Der Hauptvorteil liegt darin, daß das gasförmige 1,2-Dichlorethan durch Wärmeaustausch nach dem erfindungsgemäßen Verfahren schnonender erwärmt wird als in der Strahlungszone des Spaltofens, in der die vergleichsweise sehr viel heißeren Brennerflammen unerwünschte Wärmespitzen erzeugen können. Mit dem neuen Verfahren können deutlich höhere Spaltumsätze gefahren werden, bei gleicher oder sogar verlängerter Laufzeit des Spaltofens, oder, wenn man den Umsatz nicht erhöhen will, ist es möglich, lediglich die Laufzeit des Spaltofens erheblich zu verlängern und weniger Nebenprodukte bei der Spaltung zu erzeugen.

Nachfolgende Beispiele und Vergleichsversuche sollen die Erfindung näher erläutern:

### Beispiel 1

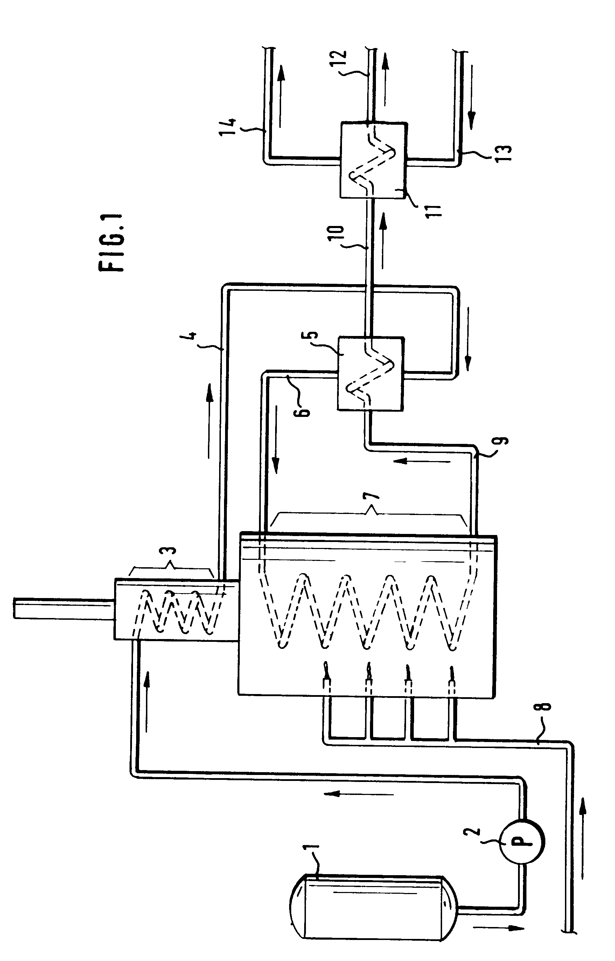

Es wird nach dem in Fig. 1 dargestellten Fließschema gearbeitet. Aus einer Pumpenvorlage (1) werden stündlich 826 kg 1,2-Dichlorethan mit einer Temperatur von 130 °C abgezogen und mit der Pumpe (2) bei einer Temperatur von 125 °C durch die Konvektionszone (3) eines Spaltofens gepumpt. Bei einer Temperatur von 260 °C verdampft 1,2-Dichlorethan vollständig, wird gasförmig aus der Konvektionszone über die Leitung (4) abgezogen, außerhalb des Ofens in einem Wärmetauscher (5) auf 350 °C erwärmt und über die Leitung (6) in die Strahlungszone (7) des Spaltofens eingeleitet. Diese Strahlungszone (7) wird durch vier übereinanderliegende Brennerreihen, denen über die Leitung (8) 0,0876 Nm³ Brennstoff (Methan) je kg erzeugtes Vinylchlorid zugeführt wird, beheizt. In der Strahlungszone (7) wird ein größerer Teil des 1,2-Dichlorethans thermisch zu Vinylchlorid und Chlorwasserstoff gespalten. Das 518 °C heiße Vinylchlorid enthaltende Gasgemisch, das die Spaltzone (7) verläßt, wird über die Leitung (9) dem Wärmetauscher (5) zugeführt und verläßt diesen über die Leitung (10) mit einer Temperatur von 433 °C. Durch die Leitung (10) gelangt das Vinylchlorid enthaltende Gas in einen weiteren Wärmetauscher (11), den es mit 220 °C über die Leitung (12) verläßt. Anschließend wird es wie üblich abgekühlt und destilliert. Der Druck, mit dem das heiße Vinylchlorid enthaltende Gas über die Leitung (9) die Strahlungszone (7) verläßt, beträgt 1,8 Mpa. Er wird am Kopf der Kolonne, in der Chlorwasserstoff abdestilliert wird, eingestellt. Dem Wärmetauscher (11)wird über die Leitung (13) Kessel-Speisewasser mit einer Temperatur von 100 °C zugeführt und daraus 90 kg/h Waserdampf mit einem Druck von 0,9 MPa und einer Temperatur von 175 °C erzeugt, dies entspricht einer Wärmemenge von 679,5 kJ/kg Vinylchlorid oder 0,0191 Nm³ Brennstoff (Methan) je 1 kg erzeugtes Vinylchlorid. Der effektive Brennstoff-(Methan)-Verbrauch reduziert sich damit auf 0,0685 Nm³ Methan je 1 kg erzeugtes Vinylchlorid. Der Wasserdampf verläßt den Wärmetauscher (11) über die Leitung (14).

Es werden 313 kg/h Vinylchlorid produziert, der Umsatz der Spaltung beträgt 60 %, die Laufzeit des Spaltofens 7 Monate.

### Vergleichsversuch A

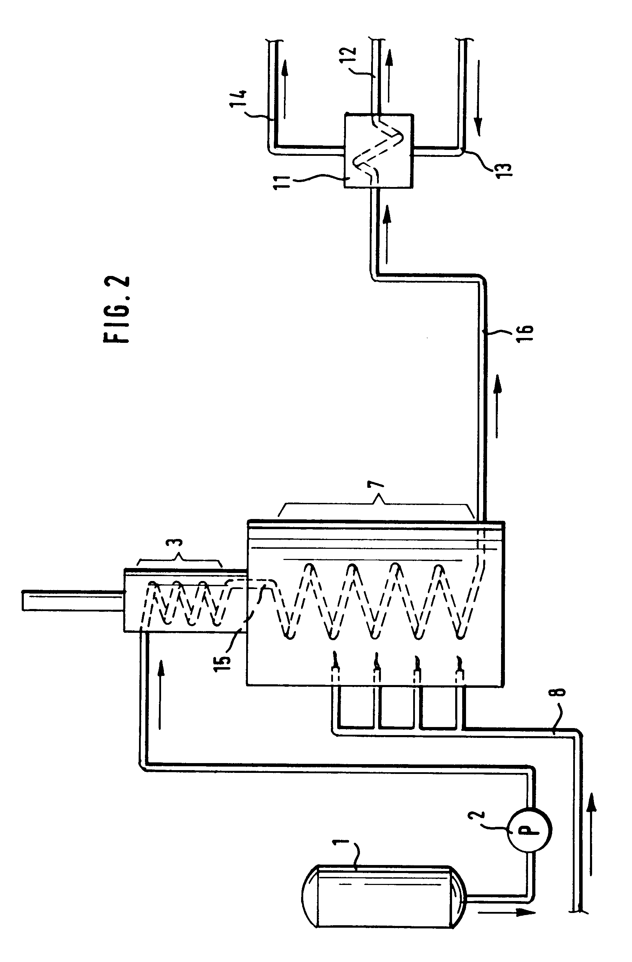

Es wird nach dem in Fig. 2 dargestellten Fließschema gearbeitet, in dem die Bezugsziffern die gleiche Bedeutung haben wie in Beispiel 1 erklärt. Abweichend von Beispiel 1 sind folgende Versuchsbedingungen: Es werden aus der Pumpenvorlage (1) über die Pumpe (2) 904 kg/h 1,2-Dichlorethan mit einer Temperatur von 125 °C in die Konvektionszone (3) des Spaltofens gepumpt, dort verdampft und gasförmig im Ofen weiter über die Leitung (15) in die Strahlungzone (7) geleitet. Aus der Strahlungszone (7) wird das heiße Vinylchlorid enthaltende Gas mit einer Temperatur von 530 °C über die Leitung (16) in den Wärmetauscher (11) geführt und verläßt diesen mit einer Temperatur von 220 °C über die Leitung (12). In den Wärmetauscher (11) wird über die Leitung (13) Kessel-Speisewasser von 100 °C geleitet und aus diesem 142 kg/h Wasserdampf mit einem Druck von 0,9 MPa und einer Temperatur von 175 °C über die Leitung (14) abgeführt. Dies entspricht einer Wärme von 1100 kJ/kg Vinylchlorid, entsprechend einer Brennstoffmenge von 0,03 Nm³ Methan je 1 kg erzeugtes Vinylchlorid. Die vier Brennerreihen im Spaltofen werden über die Leitung (8) mit 0,109 Nm³ Methan je 1 kg produziertes VC beaufschlagt. Der effektive Brennstoff-Verbrauch beträgt also 0,079 Nm³ Methan je 1 kg erzeugtes Vinylchlorid, das sind 15,3 % mehr als in Beispiel 1. Es werden 313 kg/h Vinylchlorid produziert, der Umsatz der Spaltung beträgt 55 %, die Laufzeit des Spaltofens 4 Monate. Dieser Vergleichsversuch wurde analog dem in der europäischen Patentanmeldung 21 381 beschriebenen Verfahren durchgeführt.

### Beispiel 2

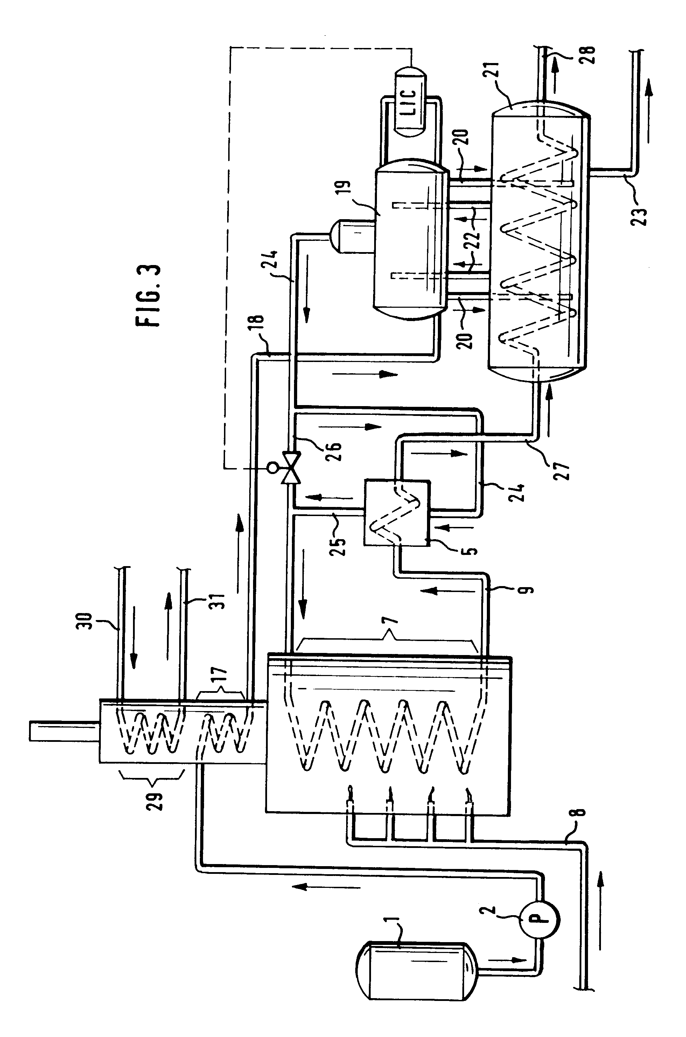

Es wird nach dem in Fig. 3 gezeigten Schema gearbeitet. Aus einer Pumpenvorlage (1) werden stündlich 798,5 kg 1,2-Dichlorethan abgezogen und mit einer Pumpe (2) mit einer Temperatur von 125 °C durch einen Teilbereich (17) der Konvektionszone eines Spaltofens gepumpt. In diesem Teilbereich (17) erwärmt sich das 1,2-Dichlorethan auf 240 °C und wird flüssig über die Leitung (18) einem Verdampfer (19) zugeführt. Aus dem Verdampfer (19) gelange es über die absteigenden Leitungen (20) in den Apparat (21) und von diesem über die aufsteigenden Leitungen (22) im Kreislauf wieder in den Verdampfer (19). Das gasförmige 1,2-Dichlorethan verläßt den Verdampfer (19) mit einer Temperatur von 260 °C und wird über die Leitung (24) einem Wärmetauscher (5) zugeführt, den es mit einer Temperatur von 367 °C über die Leitung (25) verläßt und in die Strahlungszone (7) des Spaltofens eingeleitet wird. Über einen Nebenschluß (26), der ein Regelventil enthält, welches über die Standhöhe der Flüssigkeit im Verdampfer (19) geregelt wird, kann gasförmiges 1,2-Dichlorethan vom Verdampfer (19) direkt in die Strahlungszone (7) des Spaltofens eingeleitet werden. Die Menge dieses gasförmigen 1,2-Dichlorethans ist im Vergleich zu der Menge, die durch den Wärmetauscher (5) geleitet wird, gering und dient lediglich dem Energieausgleich bei Prozeßschwankungen. Aus der Strahlungszone (7) wird das heiße, Vinylchlorid enthaltende Gas mit einer Temperatur von 523 °C über die Leitung (9) dem Wärmetauscher (5) zugeführt und verläßt diesen mit einer Temperatur von 422 °C über die Leitung (27), die in die Apparatur (21) führt, in der das flüssige 1,2-Dichlorethan, das aus dem Verdampfer (19), wie oben beschrieben, umläuft, indirekt durch das heiße Vinylchlorid enthaltende Gas bis zum Sieden erwärmt wird. Das Vinylchlorid enthaltende Gas verläßt über die Leitung (28) mit einer Temperatur von 265 °C die Apparatur (21), wird dann wie üblich abgekühlt und destillativ aufgearbeitet, wobei am Kopf der Destillationskolonne, in der Chlorwasserstoff abdestilliert wird, der Druck so eingestellt wird, daß die heißen Vinylchlorid enthaltenden Gase die Strahlungszone (7) des Spaltofens über die Leitung (9) mit einem Druck von 1,9 MPa verlassen.

Über die Leitung (23) werden aus der Apparatur (21) stündlich 28 kg 1,2-Dichlorethan, das Feststoff-Partikel enthält, abgezogen, an anderer Stelle des Prozesses von den Feststoffen befreit und wieder eingesetzt. Über die Leitung (8) werden die vier Brennerreihen des Spaltofens mit insgesamt 0,075 Nm³ Brennstoff (Methan) je 1 kg erzeugtes Vinylchlorid beaufschlagt. Im oberen Teil (29) der Konvektionszone des Spaltofens werden in einem Economizer 475 dm³/h Kessel-Speisewasser (Druck 2,5 MPa), das über die Leitung (30) mit 100 °C zugeführt wird, auf 150 °C erwärmt und über die Leitung (31) abgeführt und an anderer Stelle des Prozesses verwendet. Hierdurch werden 317,7 kJ/kg Vinylchlorid an Energie wiedergewonnen, entsprechend einer Brennstoff-(Methan)-Menge von 0,009 Nm³ je 1 kg erzeugtes Vinylchlorid. Der effektive Brennstoff-Verbrauch reduziert sich damit auf 0,066 Nm³ pro kg erzeugtes Vinylchlorid.

Es werden 330 kg/h Vinylchlorid produziert, der Umsatz bei der Spaltung beträgt 68 %, die Laufzeit des Spaltofens 12 Monate.

### Vergleichsversuch B

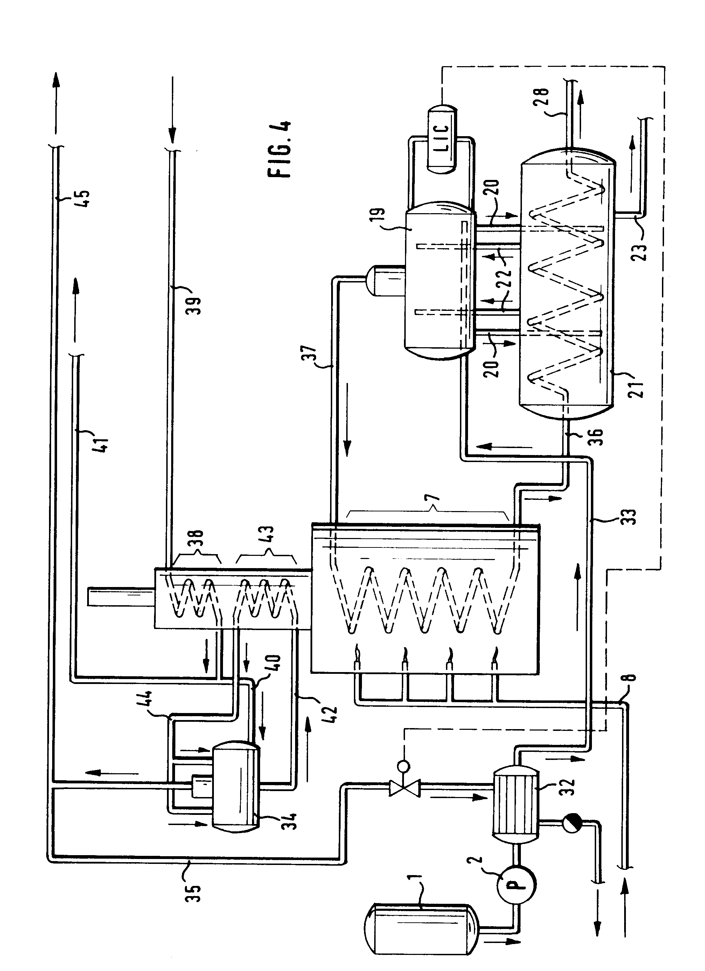

Dieser Vergleichsversuch wird analog dem Beispiel 2 der deutschen Patentanmeldung P 36 34 550 durchgeführt. Es wird nach dem in Fig. 4 dargestellten Schema gearbeitet.

Aus einer Pumpenvorlage (1) werden stündlich 834 kg 1,2-Dichlorethan abgezogen und mit der Pumpe (2) mit einer Temperatur von 125 °C über den Wärmetauscher (32) und über die Leitung (33) in den Verdampfer (19) befördert. Der Wärmetauscher (32) wird mit 25 kg/h Hockdruck-Dampf (2,1 MPa Druck; 215 °C) aus dem Kessel (34) über die Leitung (35) beheizt. Über die Messung der Standhöhe des flüssigen 1,2-Dichlorethans (LIC) im Verdampfer (19) als Regelgröße, wird die Hochdruck-Dampfzufuhr zum Wärmetauscher (32) geregelt. Das 1,2-Dichlorethan verläßt den Wärmetauscher (32) mit einer Temperatur von 161 °C.

Das heiße, vinylchloridhaltige Gas verläßt die Strahlungszone (7) des Spaltofens über die Leitung (36) mit einer Temperatur von 533 °C, durchläuft den Apparat (21) und verläßt diesen mit einer Temperatur von 245 °C über die Leitung (28). Danach wird das vinylchloridhaltige Gas nach üblichem Verfahren weiter abgekühlt und in einer Kolonne Chlorwasserstoff abdestilliert. Der Druck am Kopf dieser Kolonne wird so eingestellt, daß das heiße vinylchloridhaltige Gas die Strahlungszone (7) des Spaltofens mit einem Druck von 1,9 MPa verläßt. In den Leitungen 20 und 22 wird flüssiges 1,2-Dichlorethan, wie in Beispiel 2 beschreiben, im Kreis geführt. Das gasförmige 1,2-Dichlorethan wird über die Leitung (37) aus dem Verdampfer (19) in die Strahlungszone (7) des Spaltofens eingespeist.

Aus dem Apparat (21) werden über die Leitung (23) 30 kg/h flüssiges 1,2-Dichlorethan abgezogen, von Feststoffen befreit und an anderer Stelle des Prozesses wieder eingesetzt. Die vier übereinanderliegenden Brennerreihen des Spaltofens werden über die Leitung (8) mit insgesamt 0,1074 Nm³ Brennstoff (Methan) je 1 kg erzeugtes Vinylchlorid beaufschlagt. Im oberen Teil (38) der Konvektionszone des Spaltofens werden in einem Economizer 330 dm³ /h Kessel-Speisewasser (Druck 2,5 MPa), das über die Leitung (39) mit 80 °C zugeführt wird, auf 150 °C erwärmt und teilweise über die Leitung (40) in den Kessel (34) eingespeist, teilweise über die Leitung (41) an anderer Stelle des Vinylchlorid-Herstellungsprozesses wiederverwendet. Die Flüssigkeit aus dem Kessel (34) wird über die Leitung (42) dem unteren Teil (43) der Konvektionszone des Spaltofens zugeführt, dort erwärmt und in den Kessel (34) über die Leitung (44) eingespeist. Ein Teil des im Kessel (34) erzeugten Dampfes wird, wie oben bereits erwähnt, zur Heizung des Wärmetauschers (32) verwendet. Der größere Teil dieses Dampfes, nämlich 167 kg/h, wird über die Leitung (45) abgeführt und an anderen Stellen des Prozesses zur Erzeugung von Vinylchlorid genutzt. Hierdurch werden 1236,2 kJ/kg Vinylchlorid an Energie wiedergewonnen. Durch die Leitung (41) werden 136 dm³/h Kessel-Speisewasser mit einer Temperatur von 150 °C der weiteren Verwendung zugeführt, wodurch 121 kJ/kg Vinylchlorid an Energie zurückgewonnen werden. Die gesamte wiedergewonnene Energiemenge beträgt 1236,2 + 121 = 1357,2 kJ/kg Vinylchlorid, dies entspricht einer Brennstoff-(Methan)-Menge von 0,038 Nm³/kg Vinylchlorid. Der effektive Heizgas-Verbrauch reduziert sich damit auf 0,0694 Nm³/kg, dies sind 5 % mehr als in Beispiel 2 erforderlich war. Die Spaltofen-Laufzeit beträgt 9 Monate, es werden 330 kg/h Vinylchlorid produziert, der Umsatz bei der Spaltung des 1,2-Dichlorethans beträgt 65 %.

In allen Beispielen und Vergleichsversuchen wird ein technisches 1,2-Dichlorethan eingesetzt, das 99,7 Gew.-% reines 1,2-Dichlorethan enthält, der Rest sind überliche Nebenprodukte wie Trichlorethan; Benzol; 1,1-Dichlorethan; Trichlorethylen; Tetrachlorethylen; Chloroform; Tetrachlorkohlenstoff; Chloropren.

## Patentansprüche

1. Verfahren zur Herstellung von Vinylchlorid durch thermische Abspaltung von Chlorwasserstoff aus 1,2-Dichlorethan in einem Spaltofen, der eine Strahlungszone enthält, wobei 1,2-Dichlorethan in einem Wärmetauscher mit heißem, Vinylchlorid enthaltendem Gas, das nach der thermischen Spaltung aus der Strahlungszone des Spaltofens abgeführt wird, indirekt erwärmt wird, dadurch gekennzeichnet, daß ein Gas, das mindestens 95 Gew.-% 1,2-Dichlorethan enthält, von 170 bis 280 °C auf eine Temperatur erwärmt wird, die mindestens 50 °C unter der Temperatur liegt, mit der das Vinylchlorid enthaltende Gas aus der Strahlungszone des Spaltofens abgeführt wird und anschließend das mindestens 95 Gew.-% 1,2-Dichlorethan enthaltende Gas in den Spaltofen eingespeist wird.

2. Verfahren nach Anspruch 1, wobei ein Spaltofen verwendet wird, der eine Strahlungs- und eine Konvektionszone enthält, dadurch gekennzeichnet, daß das mindestens 95 Gew.-% 1,2-Dichlorethan enthaltende Gas ganz oder teilweise in der Konvektionszone des Spaltofens durch indirekten Wärmetausch mit den Rauchgasen aus flüssigem 1,2-Dichlorethan erzeugt wurde.

3. Verfahren nach Anspruch 1, dadurch gekennzeichnet, daß das mindestens 85 Gew.-% 1,2-Dichlorethan enthaltende Gas ganz oder teilweise außerhalb des Spaltofens in einem Verdampfer aus flüssigem 1,2-Dichlorethan erzeugt wurde.

4. Verfahren nach Anspruch 3, dadurch gekennzeichnet, daß das mindestens 95 Gew.-% 1,2-Dichlorethan enthaltende Gas ganz oder teilweise aus flüssigem 1,2-Dichlorethan durch indirekten Wärmetausch mit heißem Vinylchlorid enthaltendem Gas, das nach der thermischen Spaltung des 1,2-Dichlorethans aus der Strahlungszone des Spaltofens abgeführt wurde, erzeugt wurde.

5. Verfahren nach Anspruch 4, dadurch gekennzeichnet, daß das heiße, Vinylchlorid enthaltende Gas, das nach der thermischen Spaltung aus der Strahlungszone des Spaltofens abgeführt wird, zunächst im indirekten Wärmetausch das mindestens 95 Gew.-% 1,2-Dichlorethan enthaltende Gas erwärmt und anschließend durch erneuten indirekten Wärmetausch aus flüssigem 1,2-Dichlorethan das mindestens 95 Gew.-% 1,2-Dichlorethan enthaltende Gas erzeugt.

6. Verfahren nach einem oder mehreren der Ansprüche 1 bis 5, dadurch gekennzeichnet, daß das heiße Vinylchlorid enthaltende Gas unter einem Druck von 0,5 bis 3 MPa aus der Strahlungszone des Spaltofens abgeführt wird.

## Claims

1. A process for the production of vinyl chloride by thermal elimination of hydrogen chloride from 1,2-dichloroethane in a cracking furnace which contains a radiation zone by indirectly warming 1,2-dichloroethane in a heat exchanger by means of hot vinyl chloride-containing gas, which is drawn off from the radiation zone of the cracking furnace, after pyrolysis, characterized by heating a gas containing at least 95% by weight of 1,2-dichloroethane that is warmed from between 170 and 280°C to a temperature which is at least 50°C under the temperature at which the vinyl chloride-containing gas is drawn off from the radiation zone of the cracking furnace and then feeding the gas which contains at least 95% by weight of 1,2-dichloroethane into the cracking furnace.

2. The process as claimed in claim 1, where a cracking furnace which contains a radiation and a convection zone is used, where in the gas containing at least 95% by weight of 1,2-dichloroethane was generated from liquid 1,2-dichloroethane wholly or partly in the convection zone of the cracking furnace by indirect heat exchange using the exhaust gases.

3. The process as claimed in claim 1, where in the gas containing at least 95% by weight of 1,2-dichloroethane was generated from liquid 1,2-dichloroethane wholly or partly outside the cracking furnace in an evaporator.

4. The process as claimed in claim 3, wherein the gas containing at least 95% by weight of 1,2-dichloroethane was generated from liquid 1,2-dichloroethane wholly or partly by indirect heat exchange with hot vinyl chloride-containing gas, which was drawn off from the radiation zone of the cracking furnace after pyrolysis of the 1,2-dichloroethane.

5. The process as claimed in claim 4, wherein the hot, vinyl chloride-containing gas, which is drawn off from the radiation zone of the cracking furnace after the pyrolysis, initially warms the gas containing at least 95% by weight of 1,2-dichloroethane in the indirect heat exchange and subsequently generates the gas containing at least 95% by weight of 1,2-dichloroethane from liquid 1,2-dichloroethane by another indirect heat exchange.

6. The process as claimed in one or more of claims 1 to 5, wherein the hot vinyl chloride-containing gas is drawn off from the radiation zone of the cracking furnace at a pressure of 0.5 to 3 MPa.

## Revendications

1. Procédé pour préparer du chlorure de vinyle par dissociation thermique du chlorure d'hydrogène à partir du dichloro-1,2 éthane dans un four de dissociation contenant une zone de rayonnement, le dichloro-1,2 éthane étant indirectement chauffé dans un échangeur de chaleur avec un gaz chaud contenant du chlorure de vinyle et qui, après la dissociation thermique, est évacué de la zone de rayonnement du four de dissociation, caractérisé en ce qu'on chauffe un gaz contenant au moins 95 % en poids de dichloro-1,2 éthane d'une température de 170 à 280°C à une température d'au moins 50°C inférieure à la température à laquelle le gaz contenant du chlorure de vinyle est évacué de la zone de rayonnement du four de dissociation, le gaz contenant au moins 95 % en poids de dichloro-1,2 éthane étant ensuite introduit dans le four de dissociation.

2. Procédé selon la revendication 1, dans lequel on utilise un four de dissociation contenant une zone de rayonnement et une zone de convection, caractérisé en ce que le gaz contenant au moins 95 % en poids de dichloro-1,2 éthane a été en totalité ou en partie produit dans la zone de convection du four de dissociation par échange de chaleur indirect avec les fumées provenant du dichloro-1,2 éthane liquide.

3. Procédé selon la revendication 1, caractérisé en ce que le gaz contenant au moins 95 % en poids de dichloro-1,2 éthane a été en totalité ou en partie produit à l'extérieur du four de dissociation dans un évaporateur à partir du dichloro-1,2 éthane liquide.

4. Procédé selon la revendication 3, caractérisé en ce que le gaz contenant au moins 95 % en poids de dichloro-1,2 éthane a été produit en totalité ou en partie à partir de dichloro-1,2 éthane liquide par échange de chaleur indirect avec un gaz chaud contenant du chlorure de vinyle,qui a été soutiré de la zone de rayonnement du four de dissociation après la dissociation thermique du dichloro-1,2 éthane.

5. Procédé selon la revendication 4, caractérisé en ce que le gaz chaud contenant du chlorure de vinyle et qui est soutiré de la zone de rayonnement du four de dissociation après la dissociation thermique, chauffe d'abord, dans l'échangeur de chaleur indirect, le gaz contenant au moins 95 % en poids de dichloro-1,2 éthane puis, par un nouvel échange de chaleur indirect, produit à partir du dichloro1,2 éthane liquide le gaz contenant au moins 95 % en poids de dichloro-1,2 éthane.

6. Procédé selon l'une ou plusieurs des revendications 1 à 5, caractérisé en ce que le gaz chaud contenant du chlorure de vinyle est soutiré de la zone de rayonnement du four de dissociation sous une pression de 0,5 à 3 MPa.
